Europäisches Patentamt

European Patent Office

Office européen des brevets

(19)

(11) **EP 0 405 988 B1**

(12) **EUROPEAN PATENT SPECIFICATION**

(45) Date of publication and mention
of the grant of the patent:
**27.03.1996 Bulletin 1996/13**

(51) Int Cl.6: **C12Q 1/28**, C12Q 1/26,
G01N 33/58

(21) Application number: **90307107.4**

(22) Date of filing: **28.06.1990**

(54) **Use of superoxide dismutase in assays involving an oxidase**

Verwendung von Superoxiddismutase in Untersuchungsverfahren unter Einbeziehung von einer Oxidase

Utilisation de la superoxide dismutase dans des essais impliquant une oxidase

(84) Designated Contracting States:
**AT BE CH DE DK ES FR GB GR IT LI LU NL SE**

(30) Priority: **28.06.1989 JP 166255/89**

(43) Date of publication of application:
**02.01.1991 Bulletin 1991/01**

(73) Proprietor: **Sankyo Company Limited
Tokyo (JP)**

(72) Inventors:
• **Ikegami, Takashi, c/o Sankyo Company Ltd.
Tokyo (JP)**
• **Sato, Yoshihiro, c/o Sankyo Company Ltd.
Tokyo (JP)**
• **Sekiya, Koichi, c/o Sankyo Company Ltd.
Tokyo (JP)**
• **Saito, Yukio, c/o Sankyo Company Ltd.
Tokyo (JP)**

(74) Representative:
**Gibson, Christian John Robert et al
MARKS & CLERK,
57/60 Lincoln's Inn Fields
London WC2A 3LS (GB)**

(56) References cited:
**EP-A- 0 186 134        GB-A- 2 019 562**

• **WPIL, Week 8904, Derwent Publications Ltd.,
London (GB); AN 89-029589**
• **WPIL, Week 8849, Derwent Publications Ltd.,
London (GB); AN 88-350720**
• **WPIL, Week 8630, Derwent Publications Ltd.,
London (GB); AN 86-194626**
• **CLINICA CHIMICA ACTA, vol. 81, 1977;
SCHUURS et al., pp. 1-40**
• **WPIL, Week 8120, Derwent Publications Ltd.,
London (GB); AN 81-35400D**
• **WPIL, Week 9112, Derwent Publications Ltd.,
London (GB); AN 91-083877**

## Description

The present invention relates to assays involving the detection of peroxide.

Enzyme assays may be used to detect a substrate whose reaction products may be quantitatively detected. However, such assays are of an extremely limited nature, as the enzyme is specific for only one substrate, or type of substrate. If it is a type of substrate, then there can be no way of knowing which particular substrate has been detected. If it is a single substrate, then the assay is limited to detecting that substrate and no other, so that this sort of assay cannot be generally applied, as there are few substrates which lend themselves to a specific enzyme reaction that produces a readily assayed product.

Another form of assay is the immunoassay, which involves generating antibodies, preferably monoclonal antibodies for greater specificity, to bind the target antigen. The assay then involves some method of assaying bound antibody, and the most accurate to date has been to label the antibody with a radioisotope, or else to label another antibody directed against either the first antibody or the antigen, in a similar manner. Fluorescent labelling of one of the antibodies is one known technique. Among other problems, techniques which involve labelling of the antibody frequently require assaying the label in situ.

Radioimmunoassays additionally suffer from other disadvantages, primarily due to the essential use of radioisotopes. The additional disadvantages include: potential radiation damage to the patient; risk of environmental pollution; the requirement for special devices or facilities for prevention of radioisotope diffusion; costly apparatus; costly reagents; the necessity for qualified staff to handle radioisotopes; the general inconvenience of treatment; and transportation or preservation of the radioisotopes, due to their general instability.

Alternative labels would provide a great advantage over radioisotopes, provided that such labels were of comparable sensitivity.

Among the candidates for alternative labels is a joint enzyme and immuno- assay. An enzyme immunoassay has the potential to be a highly sensitive and powerful diagnostic technique. The technique is based both on the antigen-antibody reaction, which in itself is very specific and which still occurs even when there are only small amounts of antigen, and also on the great sensitivity and specificity of the enzyme/substrate catalytic interaction, but without the restriction of being limited to a particular substrate for the enzyme. The antibody binds the relevant antigen, which need not be an enzyme substrate at all, and the enzyme, which is linked to the antibody, is allowed to react with its own substrate, after excess antibody has been removed.

Enzyme immunoassays have now been developed to the extent that their sensitivity and accuracy are frequently equal to or higher than that of radioimmunoassays.

The potential advantages of enzyme immunoassays are great by comparison to radioimmunoassays. Simple and rapid determination is possible, and the technique does not require separation of the analytical sample, even when it is a biological sample containing complex components.

However, a major drawback is that enzymes, especially intracellular enzymes, are products of nature designed to function in a highly regulated environment, where resistance to denaturing, or otherwise deleterious factors, is either totally unnecessary, or is not a prime requisite. Further, this highly regulated environment is also highly complex and, although it is usually possible to ascertain which components are the most important in the regulation and activity of any one enzyme, the totality of the factors affecting the enzyme can only be deduced empirically.

Thus, the usefulness of enzymes from the point of view of sensitivity is tempered by the delicacy with which they must be handled and the conditions under which they will operate. For example, the desired enzyme may be somewhat unstable and subject to inactivation when used in enzyme immunoassay, and its activity may be affected dramatically by such factors as pH, temperature, reaction time and other factors, all of which may interfere with the reaction system, and any or all of which may affect the results of the assay and which, if not catered for, can lead to unpredictable and unrepeatable results being obtained. Thus, the usefulness of the technique may be severely compromised.

Nevertheless, enzyme immunoassays have been carefully developed and, in skilled hands, are generally reliable.

Absorption spectrophotometry is the method most frequently employed for the determination of enzyme activity in enzyme immunoassays. If high sensitivity is required, fluorimetry or chemiluminescence methods may be employed. However, when using such sensitive methods, the greater sensitivity can often lead to serious problems being encountered in obtaining accurate and reliable luminescent intensity readings, integrated over time, for the base solution of enzymatic substrate (reagent blank) at low concentrations.

The reliable detection limit at low concentrations is determined by the ability to extrapolate the curve generated by higher readings into these regions, or else to take reliable measurements at these concentrations. Neither method is possible where the degree of variation in the background cannot be predicted from one system to another, or where an unknown feature of the background swamps the low readings. The particular problem with the latter instance is one of quantity. In general, where readings are taken of substances occurring in relatively high concentrations, there will not be many factors present in sufficient quantity to affect the reading. Any such factors as may be able to affect the readings will usually be readily identifiable, and can either be eliminated from the system, or allowances built in to the readings. Making allowances is the less desirable of the two, as the presence of a large amount of an adverse factor will greatly complicate readings of the desired substrate when the substrate is present in only low concentrations.

The problem is vastly more complicated when deleterious factors only exert a noticeable effect at low substrate concentrations. In the systems concerned, when the assay is more sensitive, smaller quantities of deleterious factor are required to obscure low readings, so that the range of factors that might have an adverse effect on low readings becomes virtually infinite.

Members of the oxidase group of enzymes are useful in labelling, because they produce hydrogen peroxide as a result of their interaction with the substrate. The resultant hydrogen peroxide may be determined by, for example, chemiluminescence, but the reliability of the results, especially at low concentrations, may be severely compromised, owing to the background variation of the integrated luminescent intensity of the reagent blank, which can vary from preparation to preparation with little or no predictability, being high on one occasion and low on another occasion all because, for example, it is clear one day and rainy the next. The resulting scatter can seriously hamper research and analysis, and has limited the use of oxidase-labelled immunoassays.

Attempts to establish the cause of background variation in oxidase-labelled immunoassays, so to improve calibration at low concentrations, have so far proven fruitless. Reaction conditions and components, such as distilled water, reagents and reaction containers have all been varied and analysed, and a nitrogen atmosphere has been tried, with no appreciable effect.

EP 186 134 discloses that addition of SOD when performing methods for determining a substance involving an oxidase which catalyses the production of hydrogen peroxide and a chromogenic substrate (for the purpose of colourimetric determination) results in enhanced sensitivity and reliability.

Despite all attempts at effecting control of conditions, especially in measurement systems employing an oxidase, large variation in the reagent blank value remains a problem.

We have now discovered that a major cause of variability of readings at low concentrations of peroxide is the presence or absence of the superoxide anion, $O_2^-$.

Accordingly, the present invention provides, in a first aspect, an oxidase assay system, wherein any background superoxide has been reduced or eliminated. Reduction or elimination of superoxide is preferably achieved by the use of the enzyme superoxide dismutase, which has a particularly surprising enhancing effect on the sensitivity of the assays of the invention when peroxide is present at only low concentrations. The oxidase is not used in conjunction with a chromogenic substrate.

One advantage of the present invention lies, for example, in the provision of an oxidase assay system which is accurately and reliably sensitive to low levels of substrate. Another advantage lies in the provision of an enzyme assay system wherein background levels of activity are reduced to allow greater sensitivity of detection.

A further advantage lies in the provision of oxidase assay systems with enhanced sensitivity which use only cheap and readily available components. In addition, the enzyme immunoassays of the present invention have enhanced sensitivity and are more sensitive to low levels of substrate.

Any suitable enzyme assay system may be employed for the purposes of the present invention. In particular, assay systems wherein peroxide is detected are most preferred, and the invention also provides an assay system wherein peroxide is measured and any background superoxide has been reduced or eliminated.

An assay system using essentially only an oxidase, will not generally be for highly sensitive analysis of samples, and so the use of superoxide dismutase will not generally be required for such systems, although the use of superoxide dismutase may be of assistance in such cases, and forms a part of the present invention. Accordingly, it is most preferred to apply the present invention to enzyme immunoassays employing an oxidase label, as these are frequently used in conditions where high sensitivity is important.

The phrase "background superoxide" refers to that superoxide present in any component of the assay system which may interfere with the assay, for example, which may affect the reading of peroxide. The relevant component will generally be the sample, although superoxide may occur in one or more other components, depending on the system used, and it may be advantageous to add superoxide dismutase to any or all fractions or components in order to obtain the best results.

Superoxide radicals can be formed not only by various kinds of enzymes in vivo but also by radiation, such as ultraviolet or visible light. In high sensitivity enzyme immunoassays, for example, superoxide radicals may be formed by contact between the substrate and oxygen, when the substrate solution is prepared. Although we do not wish to be bound by theory, it is possible that the superoxide radicals react with the luminescence reagent to give high readings, making the detection limit at low concentrations subject to increased variability.

Superoxide may be reduced or eliminated by any appropriate method known in the art, provided that it does not adversely affect the reagents or leaves residues that substantially affect the assay results. Prime among these is the use of superoxide dismutase, an enzyme produced by most organisms. Superoxide dismutase is readily available and cheap to produce. For the purposes of this specification, reference to superoxide dismutase also includes reference to any other suitable superoxide-removing agent as appropriate.

Superoxide dismutase is particularly preferred for use with the present invention, as it has a surprisingly high enhancing effect on luminescent readings, making accurate detection of peroxide possible at an order of magnitude or more lower than was possible in the art, reducing the margin of error and making the linearity of the calibration curve at low concentrations considerably more reliable. Thus, the present invention allows greater accuracy of measurement at low concentrations.

Superoxide dismutase may be added at any suitable stage to the assay system, but is preferably added to the sample before the assay. Superoxide dismutase may be added to the enzyme-labeled component, but this will generally allow the enzyme less time to act to reduce superoxide levels before levels of peroxide are measured. It will be appreciated that superoxide dismutase may be added to any component of the system in addition to being added to the sample. For best results, the only particular requirement is that superoxide dismutase should be used in such a way that superoxide can be reduced or eliminated before peroxide is measured.

Where reference is made herein to peroxide and the measurement thereof, it will be understood that this reference is to any system to which the present invention may be applied. In the preferred systems, an enzyme is employed which catalyses a reaction leading to the production of specified levels of peroxide which can be assayed to give a quantitative indication of the amount of substance to be assayed.

Owing to the greatly increased sensitivity afforded by the reduction in superoxide, the present invention is particularly suitable for use with enzyme immunoassay systems, which have previously only been limited in their sensitivity by the unreliability of the calibration curves at low concentrations.

It is not known how superoxide dismutase makes such a drastic difference to the calibration curve at low concentrations, but this provides an important aspect of the present invention. Not only have we discovered that superoxide has a deleterious effect on the background reading for peroxide assay systems, but we have also found that the effect seems to be disproportionate to the quantities concerned.

As is shown in the Examples hereinafter, the background reading of a reagent sample may be, say, 1 $\mu$Asec but, after addition of superoxide dismutase, this can drop to about 0.07 $\mu$Asec. As superoxide dismutase disproportionates superoxide to yield molecular oxygen and assayable peroxide, it might be expected that, on treatment with superoxide dismutase, the background reading might drop by as much as 50% (assuming no original peroxide), but no further. As it is, the background reading can level out substantially below this, and drop to as low as 1/30 of the original reading, or even further. Thus, the effect of superoxide dismutase is particularly surprising, and would not have been expected to provide such a useful tool in the assays of the invention. It is possible that the effect of superoxide dismutase comes about because superoxide is being continually produced and so the enzyme is not functioning in a one-off capacity, but this explanation seems unlikely.

By 'reducing or eliminating' is meant causing a reduction in the background level of superoxide prior to or during measurement of peroxide. The reduction may be complete (elimination) but will generally be in the range of about 25-98%, preferably about 50-96%, and more preferably about 65-93% lower than the background level of superoxide. Levels outside of the broadest range tend not to be useful for practical purposes. If superoxide is not reduced by at least about 25%, then the increased accuracy of the assay is unlikely to be of great statistical significance and so not worth any extra expenditure of effort or materials. Reduction by greater than about 98% involves increasing amounts of superoxide dismutase to increasingly little effect, although it will be appreciated that reduction by 100% is technically desirable.

If the substrate solution with added superoxide dismutase is stored in a cool, dark place, the blank value will generally remain effectively stable and the solution remains useable for up to 12 months or more.

The present invention also provides an assay technique as described above wherein background superoxide is reduced or eliminated. The technique is as described in claims 11 to 13 hereinafter.

In particular, the invention provides an enzyme immunoassay technique which is stabilised and sensitised by the addition of superoxide dismutase. Preferably, the determination system involves antigen-antibody binding and is assayed quantitatively using a labelling enzyme.

Any conventional procedures may be employed for the enzyme immunoassay itself. Suitable examples include the procedures wherein: the substance to be assayed (antigen) may be labelled with an oxidase enzyme, then the labelled antigen, together with the antigen in the unlabelled sample, is brought into contact with the corresponding antibody, and a second antibody is used to bind the first, whereafter the immobilised enzyme can be assayed (competitive assay); and, alternatively, the antigen may be allowed to react with the corresponding immobilised antibody, and then an enzyme-labelled anti-antigen antibody is allowed to react with the system and, thereafter, assaying the enzyme (ELISA [enzyme-linked immunosorbent assay] - sandwich assay).

The ELISA technique may be conducted essentially as a 'one-step' or 'two-step' assay. The 'one-step' assay involves contacting antigen with immobilised antibody and, without washing, contacting the mixture with labeled antibody. The 'two-step' assay involves washing before contacting the mixture with labeled antibody. Other conventional methods may also be employed as suitable.

The substance to be assayed may be any substance exhibiting antigenicity, in particular, physiologically active substances existing in bodily fluids, examples of whioh include serum, plasma, urine and ascites fluid. Examples of substances in such fluids which can be assayed include various peptide and steroid hormones, foetal proteins such as $\alpha$-foetoprotein and CEA (car-

oino-embryonic antigen), immunoglobulin, antiviral antibodies and drugs.

Enzymatic labelling of the substances and/or their antibodies may be effected by conventional means (cf. Enzyme Immunoassay: published by Igaku Shoin, 1987). Such means will generally include covalent linking of the enzyme to the antigen or the antibody in question, specifically so as not to adversely affect the activity of the enzyme, by which it is meant that the enzyme must still be capable of interacting with its substrate, although it is not necessary for all of the enzyme to be active, provided that enough remains active to permit the assay to be effected. Indeed, some techniques for binding enzyme are non-specific (such as the use of formaldehyde), and may only yield a proportion of active enzyme.

It may be desirable, depending on the circumstances, to cross-link any proteins having multiple sub-units. Such cross-linking may be disadvantageous, however, from the point of view of activity, but may at least preserve some activity if the protein would otherwise dissociate.

It is not essential that the labelled antigen be the naturally occurring antigen, or at least the relevant naturally occurring antigen. As an example, if it were desired to assay for tuberculosis, then the BCG antigen would provide a useful substrate for labelling, as it is readily available and safer to use than the naturally occurring antigen.

It is usually desirable to immobilise one component of the system on a support, thereby allowing other components of the system to be brought into contact with the immobilised component and then allowing them to be readily removed without having to employ laborious and time-consuming methods. It is conceivable that a second phase may also be immobilised, but immobilisation of one phase is usually sufficient.

It is possible to immobilise the enzyme itself on a support, but if a solid-phase enzyme is required, then this is generally best achieved by binding the enzyme to an antibody and affixing the antibody to a support, models and systems for which are well-known in the art. Simple polystyrene may provide a suitable support for such antibody-bound enzymes.

Enzymes which are suitable for labelling are not particularly limited, but are selected from the members of the oxidase group. These enzymes catalyse the production of hydrogen peroxide by reaction with their substrates (suitable examples of substrates are described hereinafter). Examples of suitable enzymes include; L-amino acid oxidase, aldehyde oxidase, ethanolamine oxidase, galactose oxidase, xanthine oxidase, glycolate oxidase, glycerol oxidase, glycerol-3-phosphate oxidase, glucose oxidase. D-glutamate oxidase, cholesterol oxidase, dihydroorotate oxidase, oxalate oxidase, tyramine oxidase, L-2-hydroxylate oxidase, pyridoxine phosphate oxidase, pyruvate oxidase, putrescine oxidase, hexose oxidase, lathosterol oxidase and lysine $\alpha$-oxidase. Of these, glucose oxidase is frequently used for its stability, ease of availability and cheapness.

Activity of the enzyme employed for labelling may be assayed by measuring the concentration of hydrogen peroxide formed after reaction of the enzyme-labelled antibody with the substrate under controlled conditions by techniques well-known in the art.

Suitable substrates for the above enzymes include; L-leucine, L-methionine, L-alanine, acetaldehyde, purine, hypoxanthine, ethanolamine, D-galactose, lactose, xanthine, glycolic acid, lactic acid, glycerol, dihydroxyacetone, glycerol triphosphate, glucose, D-mannose, D-galactose, D-glutamic acid, orotic acid, D-dihydroorotic acid, oxalic acid, tyramine, dopamine, L-2-hydroxyisocapronic acid, glycolic acid, pyridoxine-5-phosphate, pyruvic acid, putrescine, D-glucose, D-galaotose, lathosterol, L-lysine, L-ornithine and L-phenylalanine. Choice will be determined according to the enzyme employed. For example, if glucose oxidase is selected, glucose is preferred.

The concentration of hydrogen peroxide may be assayed, for example, by colorimetry, fluorimetry or chemiluminesoence. When using colorimetry, appropriate reagents include 1,2-diaminobenzene or 2,2′-amino-bis(3-ethylbenzothiazoline-6-sulphonic acid). Appropriate reagents for fluorimetry include p-hydroxyphenylpropionic acid, tyramine and homovaline. For chemiluminescence, appropriate reagents include luminol, luminol derivatives, oxalate, oxalate derivatives, acridium, pyrogallol and 8-anilinonaphthalene-1-sulphonic acid. An oxidation catalyst, such as peroxidase or microperoxidase, is required before any of the above reagents can react with peroxide for the appropriate assay.

Superoxide dismutase is a well-known enzyme and has been prepared from both human and bovine erythrocytes. Recently, human recombinant superoxide dismutase has been produced in anticipation of its use as a drug. However, for the purposes of the present invention, the origin of the superoxide dismutase is of no particular relevance, and the enzyme may be prepared from any appropriate source.

The amount of superoxide dismutase used is not critical to the present invention, provided that an adequate reduction in superoxide occurs. As a general guide, a concentration of about 10 µg/ml has been found to be sufficient. Lower concentrations may be used, such as 5 µg/ml, but much lower concentrations will not reduce levels of superoxide as effectively, while higher concentrations, such as over about 20 µg/ml, have little extra effect. It has also been established that neither the formation of hydrogen peroxide nor enzymic action is affected by the presence of superoxide dismutase, so that the enzyme has no effect on an assay wherein there is no superoxide to affect the results.

All of the steps associated with the assays of the invention, such as the antigen-antibody reaction, enzyme reaction and luminescence reaction, may be conducted under conditions well-known in the art, particularly with regard to reaction time and temperature.

The invention is further illustrated herein with reference to the following Examples, which are not to be construed as limiting hereon. Although the Examples use standard solutions for ease of illustration, any standard samples, such as bodily fluids, may be used, as may other assay techniques, as described above.

EXAMPLE 1

Effect of Superoxide Dismutase on the Substrate Blank

Reagents and compositions used in the experiment were as follows:

(a) Superoxide dismutase was obtained from human recombinant culture;
(b) Substrate solution: 0.01 M acetate buffer solution (pH 5.8) containing 0.1 M glucose and 0.1% sodium azide;
(c) Chemiluminescence reagent: 100 mM CHES buffer (2-[cyclohexylamino]ethanesulphonic acid, 27.3 g dissolved in 1 litre deionised water), pH 9.5, containing 0.2 mM luminol and 60 $\mu$M microperoxidase (Dojin Chemical Laboratories);
(d) Measurement apparatus: Chemiluminescence detector (made by Sankyo, but any standard suitable spectrophotometer may be used). The luminescent intensity was calculated from the output current of the photoelectric electron-multiplier tube integrated over 10 seconds and converted to $\mu$Asec; and
(e) Test procedure: To 100 $\mu$l of substrate solution containing 0, 0.05, 0.1, 0.5, 1.5, 10 or 50 $\mu$g/ml of superoxide dismutase, 100 $\mu$l of the chemiluminescenoe reagent was added and the integrated luminescent intensity obtained as described above.

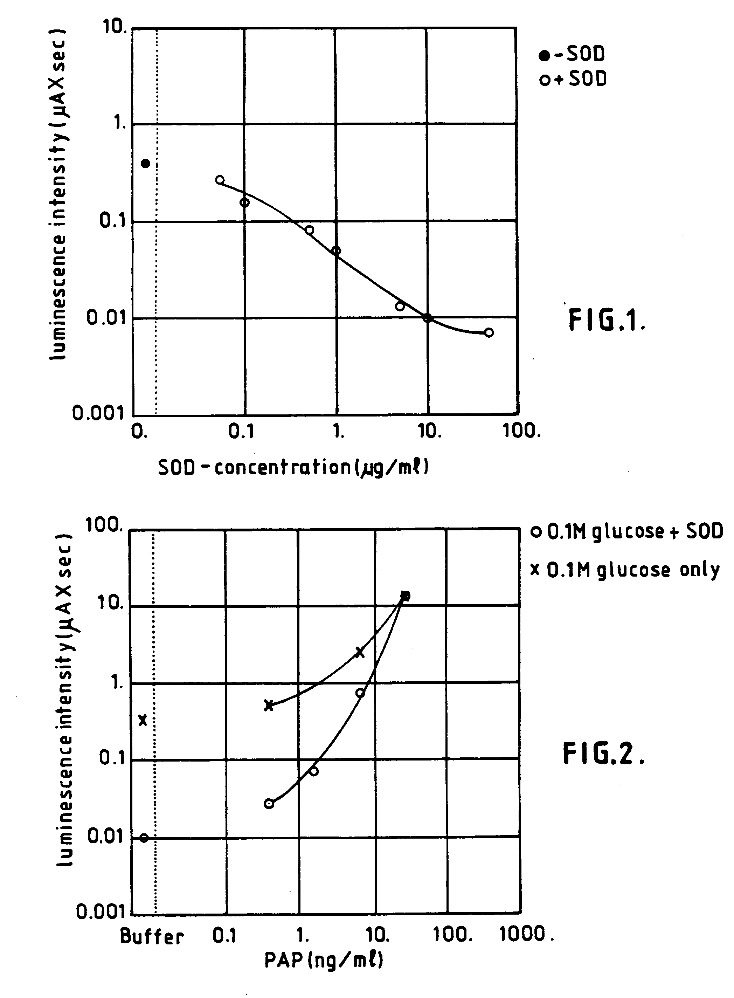

The results are shown in Figure 1, illustrating the effect of superoxide dismutase on the substrate solution, the x and y axes indicating superoxide dismutase concentration and integrated luminescence intensity respectively. The result shown by the solid circle represents the control (without superoxide dismutase).

It is readily apparent that the background readings were dramatically lowered by the addition of superoxide dismutase. Above 5 $\mu$g/ml or higher of superoxide dismutase, the lowering of the readings substantially plateaued.

The corresponding reduction in the blank value by addition of superoxide dismutase to the substrate gives rise to increased accuracy at low concentrations by allowing extrapolation of the graph obtained.

EXAMPLE 2

Calibration for Prostatic Acid Phosphatase (PAP) with Superoxide Dismutase

(a) The materials were as follows:

(i) Anti-PAP antibody coated tube.
(ii) Sample: 4 $\mu$g/ml PAP was diluted with buffer A (Na phosphate 10mM, bovine serum albumin [BSA] 0.1%, NaCl 0.1M, MgCl$_2$ 1 mM, NaN$_3$ 0.1%, made up in deionised water) to obtain 0.39, 1.56, 6.25 and 25 ng/ml of the sample solutions.
(iii) Enzyme-labelled antibody: glucose oxidase-labelled anti-PAP antibody.
(iv) Substrate:

(1) 0. 1 M glucose solution
(2) 10 $\mu$g/ml of superoxide dismutase added to substrate (1).

(b) Reaction procedure
To the anti-PAP antibody coated tube (PAP tube), 30 $\mu$l of the sample solution and 270 $\mu$l of buffer A were added. After incubation at 37°C for 15 minutes, the tube was washed with 1.5 ml of buffer A. The above procedure was repeated 3 times to effect B/F separation (separation of bound and free antibody).

Three hundred $\mu$l of glucose oxidase-labelled anti-PAP antibody was added to the PAP tube. After incubation at 37°C for 15 minutes, the tube was washed with 1.5 ml of buffer A. The procedure was repeated 3 times to effect B/F separation. Subsequently, 300 $\mu$l of the substrate were added to the PAP tube and incubated at 37°C for 15 minutes. To 100 $\mu$l of the reaction solution obtained from the tube, 100 $\mu$l of chemiluminescence reagent was added.

(c) The results are shown in Fig. 2 in the form of a calibration curve (using superoxide dismutase) for prostatic acid phosphatase (PAP).

As can clearly be seen, addition of superoxide dismutase remarkably enhanced the calibration curve at low concentrations.

EXAMPLE 3

Calibration for $\alpha$-Foetoprotein (AFP) using Superoxide Dismutase

(a) The materials used were as follows:

(i) Anti-AFP antibody coated tube;

(ii) Sample: 1080 ng/ml of AFP was diluted with buffer A to obtain 0. 54, 1.08, 10.8, 108.0 and 1080.0 ng of the sample solutions;

(iii) glucose oxidase-labelled anti-AFP antibody; and

(iv) Substrate:

(1) 0. 1 M glucose solution
(2) 0. 1 M glucose solution
containing 10 μg/ml
superoxide dismutase

(b) Reaction procedure

To the anti-AFP antibody coated tube (AFP tube), 30 μl of the sample solution and 270 μl of buffer A were added. After incubation at 37°C for 15 minutes, the tube was washed with 1.5 ml of buffer A. This procedure was repeated 3 times to effect B/F separation.

Next, 300 μl of glucose oxidase-labelled anti-AFP antibody was added to the AFP tube. After incubation at 37°C for 15 minutes, the tube was washed with 1.5 ml of buffer A. The above procedure was again repeated 3 times to effect B/F separation. Subsequently, 300 μl of the substrate was added to the AFP tube and incubated at 37°C for 15 minutes. To 100 μl of the reaction solution obtained, 100 μl of chemiluminescence reagent were added.

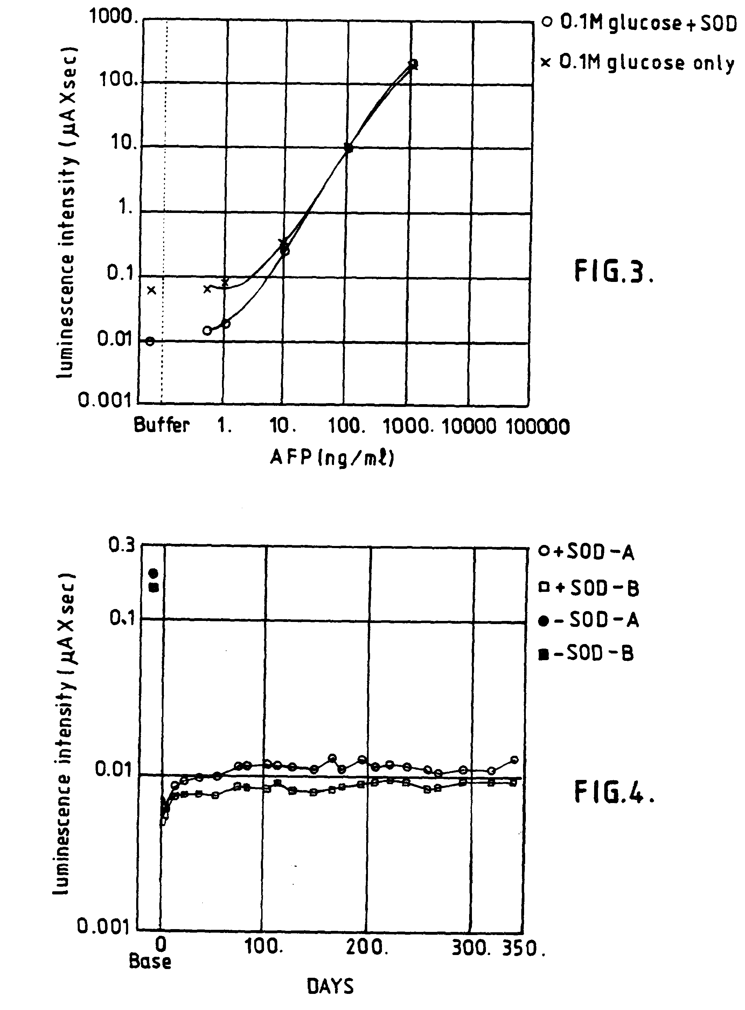

(c) The results are shown in Fig. 3 in the form of a calibration curve (using superoxide dismutase) for α-foetoprotein (AFP).

As can clearly be seen in Fig. 3, addition of superoxide dismutase dramatically improved the accuracy of low concentration assays.

EXAMPLE 4

Stability of Substrate Solution containing Superoxide Dismutase

Substrate solutions containing 10 μg/ml of added recombinant human superoxide dismutase and human erythrocyte superoxide dismutase were each put in a brown bottle and allowed to stand in a dark room at 4°C. The integrated luminescence intensities of the solutions were determined with time.

Fig. 4 shows the results obtained, illustrating the change in luminescent intensity with time for substrate solutions using superoxide dismutase from the two sources (SOD-A: human erythrocyte. SOD-B: recombinant gene. + and - mean with and without superoxide dismutase, respectively), the x and y axes representing standing time and integrated luminescence intensity respectively. The results indicated by the solid points represent the controls (no superoxide dismutase).

Thus, it can be seen that the addition of superoxide dismutase to substrate brings about a reduction in background reading to about 1/30 of the original value, irrespective of the origin of the superoxide dismutase. In the stability experiment, the background tended to rise about 10 days after the addition of superoxide dismutase, but became stable thereafter. After about 12 months, no substantial change was noted.

Thus, the use of superoxide dismutase in accordance with the present invention enhances assay accuracy at low concentrations and, in addition, the increased temporal stability of substrate solutions containing superoxide dismutase allows storage of these solutions in a useable condition over extended periods of time.

**Claims**

1. A system, preferably an enzyme immunoassay system, comprising reagents for determining an amount of a substance in a solution and comprising an oxidase which catalyses production of hydrogen peroxide in a quantity proportional to the amount of substance and means to assay the peroxide,

characterised in that the system further comprises means, preferably superoxide dismutase, to reduce levels of superoxide; provided that the oxidase is not used in conjunction with a chromogenic substrate.

2. A system according to claim 1, wherein the oxidase comprises one or more, preferably one, of: L-amino acid oxidase, aldehyde oxidase, ethanolamine oxidase, galactose oxidase, xanthine oxidase, glycolate oxidase, glycerol oxidase, glycerol-3-phosphate oxidase, glucose oxidase, D-glutamate oxidase, cholesterol oxidase, dihydroorotate oxidase, oxalate oxidase, tyramine oxidase, L-2-hydroxylate oxidase, pyridoxine phosphate oxidase, pyruvate oxidase, putrescine oxidase, hexose oxidase, lathosterol oxidase and lysine α-oxidase, preferably glucose oxidase.

3. An enzyme immunoassay system for determining an amount of an antigen in a solution, wherein an oxidase is capable of catalysing production of hydrogen peroxide from a substrate, the system comprising the substrate and means for assaying peroxide,

characterised in that the system further comprises means, preferably superoxide dismutase, to reduce levels of superoxide; provided that the oxidase is not used in conjunction with a chromogenic substrate.

4. A system according to claim 3, wherein the oxidase comprises one or more, preferably one, of: L-amino acid oxidase, aldehyde oxidase, ethanolamine oxidase, galactose oxidase, xanthine oxidase, glycolate oxidase, glycerol oxidase, glycerol-3-phosphate oxidase, glucose oxidase, D-glutamate oxidase, cholesterol oxidase, dihydroorotate oxidase, oxalate oxidase, tyramine oxidase, L-2-hydroxylate oxidase, pyridoxine phosphate oxidase, pyruvate oxidase, putrescine oxidase, hexose oxidase, lathosterol oxidase and lysine $\alpha$-oxidase,
preferably glucose oxidase.

5. A system according to claim 4, wherein the enzyme substrate comprises one or more, preferably one, of: L-leucine, L-methionine, L-alanine, acetaldehyde, purine, hypoxanthine, ethanolamine, D-galactose, lactose, xanthine, glycolic acid, lactic acid, glycerol, dihydroxyacetone, glycerol triphosphate, glucose, D-mannose, D-galactose, D-glutamic acid, orotic acid, D-dihydroorotic acid, oxalic acid, tyramine, dopamine, L-2-hydroxyisocapronic acid, glycolic acid, pyridoxine-5-phosphate, pyruvic acid, putrescine, D-glucose, D-galactose, lathosterol, L-lysine, L-ornithine and L-phenylalanine,
preferably glucose.

6. A system according to any of claims 3 to 5, wherein the system comprises a sandwich type assay or a competitive type assay.

7. A system according to any of claims 3 to 6, wherein the superoxide reducing means is added to a solution containing the substance prior to the assay.

8. A system according to any of claims 3 to 7, wherein the superoxide reducing means is superoxide dismutase, the superoxide dismutase being present in an amount greater than about 5 $\mu$g/ml, preferably greater than about 10 $\mu$g/ml.

9. A system according to any of claims 3 to 8, wherein the enzyme is immobilised on a support.

10. A system according to claim 9, wherein the enzyme is linked to an antibody immobilised on the support.

11. A method for determining an amount of an antigen in a solution by using a system as defined in claim 1, comprising:

   (i) contacting the antigen with an antibody therefor immobilised on a support;
   (ii) removing free antigen;
   (iii) contacting bound antigen with oxidase-labeled antibody capable of binding the bound antigen;
   (iv) removing free oxidase-labeled antibody;
   (v) introducing a substrate for the oxidase so as to permit assaying of enzyme activity thereby,
   and optionally repeating at least one of the above steps at least once, the oxidase being capable of catalysing production of peroxide from the substrate, characterised in that the method further comprises use of means, preferably superoxide dismutase, for reducing levels of superoxide in at least one of the steps (i) to (v).

12. A method for determining an amount of an antigen in a solution by using a system as defined in claim 1, comprising:

   (i) contacting the antigen and an amount of the antigen labeled with an oxidase with an antibody therefor to form a complex;
   (ii) contacting the complex with an immobilised antibody capable of binding the complex;
   (iii) removing free complex;
   (iv) introducing a substrate for the oxidase so as to permit assaying of enzyme activity thereby,
   and optionally repeating at least one of the above steps at least once, the oxidase being capable of catalysing production of peroxide from the substrate, characterised in that the method further comprises use of means, preferably superoxide dismutase, for reducing levels of superoxide in at least one of the steps (i) to (iv).

13. A method for determining an amount of an antigen in a solution by using a system as defined in claim 1, comprising:

   (i) contacting the antigen with an antibody therefor immobilised on a support;
   (ii) contacting bound antigen with oxidase-labeled antibody capable of binding the bound antigen;
   (iii) removing free oxidase-labeled antibody, free antigen and free antigen/enzyme-labeled antibody complex;
   (iv) introducing a substrate for the oxidase so as to permit assaying of enzyme activity thereby,
   and optionally repeating at least one of the above steps at least once, the oxidase being capable of catalysing production of peroxide from the substrate, characterised in that the method further comprises use of means, preferably superoxide dismutase, for reducing levels of superoxide in at least one of the steps (i) to (iv).

14. A method according to any of claims 11 to 13, wherein the oxidase comprises one or more, preferably one, of: L-amino acid oxidase, aldehyde oxidase, ethanolamine oxidase, galactose oxidase, xanthine oxidase, glycolate oxidase, glycerol oxidase, glycerol-3-phosphate oxidase, glucose oxidase, D-glutamate oxidase, cholesterol oxidase, dihydroorotate oxidase, oxalate oxidase, tyramine oxidase, L-2-hydroxylate oxidase, pyridoxine phosphate oxidase, pyruvate oxidase, putrescine oxidase, hexose oxidase, lathosterol oxidase and lysine α-oxidase,

preferably glucose oxidase.

15. A method according to claim 14, wherein the enzyme substrate comprises one or more, preferably one, of: L-leucine, L-methionine, L-alanine, acetaldehyde, purine, hypoxanthine, ethanolamine, D-galactose, lactose, xanthine, glycolic acid, lactic acid, glycerol, dihydroxyacetone, glycerol triphosphate, glucose, D-mannose, D-galactose, D-glutamic acid, orotic acid, D-dihydroorotic acid, oxalic acid, tyramine, dopamine, L-2-hydroxyisocapronic acid, glycolic acid, pyridoxine-5-phosphate, pyruvic acid, putrescine, D-glucose, D-galactose, lathosterol, L-lysine, L-ornithine and L-phenylalanine,

preferably glucose.

16. A method according to any of claims 11 to 15, wherein the superoxide reducing means is added before the substrate is added.

17. A method according to claim 16, wherein the superoxide reducing means is added to the solution containing the antigen.

18. A method according to any of claims 11 to 17, wherein the superoxide reducing means is superoxide dismutase, the superoxide dismutase being added in an amount greater than about 5 μg/ml, preferably greater than about 10 μg/ml.

19. A method according to any of claims 11 to 18, wherein the superoxide reducing means is used in preparing a reagent blank.

20. A method for sensitising an assay wherein low levels of hydrogen peroxide generated from a non-chromogenic substrate by an oxidase are detected, comprising using a means, preferably superoxide dismutase, for reducing levels of superoxide.

**Patentansprüche**

1. System, vorzugsweise Enzymimmunoassaysystem, enthaltend Reagenzien zur Bestimmung einer Substanzmenge in einer Lösung und enthaltend eine Oxidase, die die Herstellung von Wasserstoffperoxid in einer der Substanzmenge proportionalen Menge katalysiert, und Mittel, um das Peroxid zu bestimmen,

dadurch gekennzeichnet, daß das System des weiteren Mittel enthält, vorzugsweise Superoxiddismutase, um den Gehalt an Superoxid zu reduzieren;
mit der Maßgabe, daß die Oxidase nicht in Verbindung mit einem chromogenen Substrat verwendet wird.

2. System gemäß Anspruch 1, wobei die Oxidase eine oder mehrere, vorzugsweise eine, der folgenden Komponenten enthält: L-Aminosäure-Oxidase, Aldehyd-Oxidase, Ethanolamin-Oxidase, Galactose-Oxidase, Xanthin-Oxidase, Glycolat-Oxidase, Glycerin-Oxidase, Glycerin-3-phosphat-Oxidase, Glucose-Oxidase, D-Glutamat-Oxidase, Cholesterin-Oxidase, Dihydroorotat-Oxidase, Oxalat-Oxidase, Tyramin-Oxidase, L-2-Hydroxylat-Oxidase, Pyridoxinphosphat-Oxidase, Pyruvat-Oxidase, Putrescin-Oxidase, Hexose-Oxidase, Lathosterin-Oxidase und Lysin-α-Oxidase,

vorzugsweise Glucose-Oxidase.

3. Enzymimmunoassaysystem zur Bestimmung einer Antigenmenge in einer Lösung, wobei eine Oxidase zur Katalyse der Bildung von Wasserstoffperoxid aus einem Substrat befähigt ist, und das System das Substrat und Mittel zur Bestimmung des Peroxids enthält,

dadurch gekennzeichnet, daß das System des weiteren Mittel enthält, vorzugsweise Superoxiddismutase, um den Gehalt an Superoxid zu reduzieren;
mit der Maßgabe, daß die Oxidase nicht in Verbindung mit einem chromogenen Substrat verwendet wird.

4. System gemäß Anspruch 3, in dem die Oxidase eine oder mehrere, vorzugsweise eine, der folgenden Komponenten umfaßt: L-Aminosäure-Oxidase, Aldehyd-Oxidase, Ethanolamin-Oxidase, Galactose-Oxidase, Xanthin-Oxidase, Glycolat-Oxidase, Glycerin-Oxidase, Glycerin-3-phosphat-Oxidase, Glucose-Oxidase, D-Glutamat-Oxidase, Cholesterin-Oxidase, Dihydroorotat-Oxidase, Oxalat-Oxidase, Tyramin-Oxidase, L-2-Hydroxylat-Oxidase, Pyridoxinphosphat-Oxidase, Pyruvat-Oxidase, Putrescin-Oxidase, Hexose-Oxidase, Lathosterin-Oxidase und Lysin-α-Oxidase,

vorzugsweise Glusose-Oxidase.

5. System gemäß Anspruch 4, wobei das Enzymsubstrat eine oder mehrere, vorzugsweise eine, der folgenden Komponenten umfaßt: L-Leucin, L-Methionin, L-Alanin, Acetaldehyd, Purin, Hypoxanthin, Ethanolamin, D-Galactose, Lactose, Xanthin, Glycolsäure, Milchsäure, Glycerin, Dihydroxyaceton, Glycerintriphosphat, Glucose, D-Mannose, D-Galactose, D-Glutaminsäure, Orotsäure, D-Dihydroorotsäure, Oxalsäure, Tyramin, Dopamin, L-2-Hydroxyisocapronsäure, Glycolsäure, Pyridoxin-5phosphat, Brenztraubensäure, Putrescin, D-Glucose, D-Galactose, Lathosterin, L-Lysin, L-Ornithin und L-Phenylalanin,
vorzugsweise Glucose.

6. System gemäß einem der Ansprüche 3 bis 5, wobei das System einen sandwichartigen Assay oder einen kompetitiven Assay beinhaltet.

7. System gemäß einem der Ansprüche 3 bis 6, wobei das Mittel, welches das Superoxid reduziert, vor dem Assay zu einer die Substanz enthaltenden Lösung gegeben wird.

8. System gemäß einem der Ansprüche 3 bis 7, wobei das Superoxid-reduzierende Mittel Superoxiddismutase ist, und die Superoxiddismutase in einer Menge von mehr als ungefähr 5 μg/ml, vorzugsweise mehr als etwa 10 μg/ml, vorhanden ist.

9. System gemäß einem der Ansprüche 3 bis 8, wobei das Enzym auf einem Träger immobilisiert ist.

10. System gemäß Anspruch 9, wobei das Enzym an einem Antikörper gebunden ist, der auf dem Träger immobilisiert ist.

11. Verfahren zur Bestimmung einer Antigenmenge in einer Lösung unter Verwendung des in Anspruch 1 definierten Systems, welches beinhaltet:

   (i) Kontaktieren des Antigens mit einem dagegen gerichteten Antikörper, der auf einem Träger immobilisiert ist;
   (ii) Entfernen des freien Antigens;
   (iii) Kontaktieren des gebundenen Antigens mit Oxidasemarkiertem Antikörper, der das gebundene Antigen binden kann;
   (iv) Entfernen des freien Oxidase-markierten Antikörpers;
   (v) Zugeben eines Oxidasesubstrates, um dadurch die enzymatische Aktivität bestimmen zu können,
   und gegebenenfalls die mindestens einmalige Wiederholung von mindestens einem der oben genannten Schritte, wobei die Oxidase die Bildung von Peroxid aus dem Substrat katalysieren kann, dadurch gekennzeichnet, daß das Verfahren des weiteren die Verwendung von Mitteln, vorzugsweise Superoxiddismutase, zur Reduzierung des Gehalts an Superoxid in mindestens einem der Schritte (i) bis (v) beinhaltet.

12. Verfahren zur Bestimmung einer Antigenmenge in einer Lösung unter Verwendung des in Anspruch 1 definierten Systems, welches beinhaltet:

   (i) Kontaktieren des Antigens und einer Menge an Antigen, das mit einer Oxidase markiert ist, mit einem dagegen gerichteten Antikörper, um einen Komplex zu bilden;
   (ii) Kontaktieren des Komplexes mit einem immobilisierten Antikörper, der den Komplex binden kann;
   (iii) das Entfernen des freien Komplexes;
   (iv) Zugeben eines Oxidasesubstrates, um dadurch die enzymatische Aktivität bestimmen zu können,
   und gegebenenfalls das mindestens einmalige Wiederholen von mindestens einem der oben genannten Schritte, wobei die Oxidase die Bildung des Peroxids aus dem Substrat katalysieren kann, dadurch gekennzeichnet, daß die Methode des weiteren die Verwendung von Mitteln, vorzugsweise Superoxiddismutase, zur Reduzierung des Gehalts an Superoxid in mindestens einem der Schritte (i) bis (iv) beinhaltet.

13. Verfahren zur Bestimmung einer Antigenmenge in einer Lösung unter Verwendung des in Anspruch 1 definierten Systems, welches beinhaltet:

   (i) Kontaktieren des Antigens mit einem dagegen gerichteten Antikörper, der an einem Träger immobilisiert ist;
   (ii) Kontaktieren des gebundenen Antigens mit einem Oxidase-markierten Antikörper, der das gebundene Antigen binden kann;
   (iii) Entfernen des freien Oxidase-markierten Antikörpers, des freien Antigens und des freien Komplexes aus Antigen und Enzym-markiertem Antikörper;
   (iv) Zugeben eines Oxidasesubstrats, um dadurch die enzymatische Aktivität bestimmen zu können,
   und gegebenenfalls das mindestens einmalige Wiederholen von mindestens einem der oben genannten Schritte, wobei die Oxidase die Herstellung von Peroxid aus dem Substrat katalysieren kann, dadurch gekennzeichnet, daß die Methode des weiteren die Verwendung von Mitteln, vorzugsweise Superoxiddismutase, zur Reduzierung des Gehalts an Superoxid in mindestens einem der Schritte (i) bis (iv) beinhaltet.

14. Verfahren gemäß einem der Ansprüche 11 bis 13, wobei die Oxidase mindestens eine oder mehrere, vorzugsweise eine, der folgenden Komponenten umfaßt: L-Aminosäure-Oxidase, Aldehyd-Oxidase, Ethanolamin-Oxidase, Galactose-Oxidase, Xanthin-Oxidase, Glycolat-Oxidase, Glycerin-Oxidase, Glycerin-3-phosphat-Oxidase, Glucose-Oxidase, D-Glutamat-Oxidase, Cholesterin-Oxidase, Dihydroorotat-Oxidase, Oxalat-Oxidase, Tyramin-Oxidase, L-2-Hydroxylat-Oxidase, Pyridoxinphosphat-Oxidase, Pyruvat-Oxidase, Putrescin-Oxidase, Hexose-Oxidase, Lathosterin-Oxidase und Lysin-α-Oxidase,

vorzugsweise Glusose-Oxidase.

15. Verfahren gemäß Anspruch 14, wobei das Enzymsubstrat eine oder mehrere, vorzugsweise eine, der folgenden Komponenten umfaßt: L-Leucin, L-Methionin, L-Alanin, Acetaldehyd, Purin, Hypoxanthin, Ethanolamin, D-Galactose, Lactose, Xanthin, Glycolsäure, Milchsäure, Glycerin, Dihydroxyaceton, Glycerintriphosphat, Glucose, D-Mannose, D-Galactose, D-Glutaminsäure, Orotsäure, D-Dihydroorotsäure, Oxalsäure, Tyramin, Dopamin, L-2-Hydroxyisocapronsäure, Glycolsäure, Pyridoxin-5-phosphat, Brenztraubensäure, Putrescin, D-Glucose, D-Galactose, Lathosterin, L-Lysin, L-Ornithin und L-Phenylalanin,

vorzugsweise Glucose.

16. Verfahren gemäß einem der Ansprüche 11 bis 15, wobei das Superoxid-reduzierende Mittel vor dem Substrat zugegeben wird.

17. Verfahren gemäß Anspruch 16, wobei das Superoxid-reduzierende Mittel zu der Lösung gegeben wird, die das Antigen enthält.

18. Verfahren gemäß einem der Ansprüche 11 bis 17, wobei das Superoxid-reduzierende Mittel Superoxiddismutase ist, und die Superoxiddismutase in einer Menge von mehr als etwa 5 μg/ml, vorzugsweise mehr als etwa 10 μg/ml, zugegeben wird.

19. Verfahren gemäß einem der Ansprüche 11 bis 18, wobei das Superoxid-reduzierende Mittel dazu verwendet wird, um einen Nullwert des Reagenzes zu ermitteln.

20. Verfahren zur Sensibilisierung eines Assays, wobei geringe Konzentrationen an Wasserstoffperoxid, welche von nicht-chromogenen Substraten durch eine Oxidase gebildet werden, nachgewiesen werden, wobei dieses Verfahren die Verwendung von Mitteln, vorzugsweise Superoxiddismutase, zur Reduzierung des Gehalts an Superoxid beinhaltet.

**Revendications**

1. Système, de préférence système de dosage immuno-enzymatique, comprenant des réactifs pour la détermination d'une quantité d'une substance en solution et comprenant une oxydase qui catalyse la production de peroxyde d'hydrogène dans une quantité proportionnelle à la quantité de substance et des moyens de dosage du peroxyde,

caractérisé en ce que le système comprend en plus des moyens, de préférence la superoxyde dismutase, pour réduire les niveaux de superoxyde;
pourvu que l'oxydase ne soit pas utilisée conjointement avec un substrat chromogène.

2. Système selon la revendication 1, dans lequel l'oxydase contient une ou plusieurs, de préférence une, des enzymes suivantes : la L-amino-acide oxydase, l'adléhyde oxydase, l'éthanolamine oxydase, la galactose oxydase, la xanthine oxydase, la glycolate oxydase, la glycérol oxydase, la glycérol-3-phosphate oxydase, la glucose oxydase, la D-glutamate oxydase, la cholestérol oxydase, la dihydro-orotate oxydase, l'oxalate oxydase, la tyramine oxydase, la L-2-hydroxylate oxydase, la pyridoxine phosphate oxydase, la pyruvate oxydase, la putrescine oxydase, l'hexose oxydase, la lathostérol oxydase et la lysine α-oxydase,

de préférence la glucose oxydase.

3. Système immuno-enzymatique pour la détermination d'une quantité d'antigène dans une solution, dans lequel une oxydase est capable de catalyser la production de peroxyde d'hydrogène à partir d'un substrat, le système comprenant le substratetdes moyens de dosage du peroxyde,

caractérisé en ce que le système comprend en plus des moyens, de préférence la superoxyde dismutase, pour réduire les niveaux de superoxyde;
pourvu que l'oxydase ne soit pas utilisée conjointement avec un substrat chromogène.

4. Système selon la revendication 3, dans lequel l'oxydase contient une ou plusieurs, de préférence une, des enzymes suivantes : la L-amino-acide oxydase, l'adléhyde oxydase, l'éthanolamine oxydase, la galactose oxydase, la xanthine oxydase, la glycolate oxydase, la glycérol oxydase, la glycérol-3-phosphate oxydase, la glucose oxydase, la D-glutamate oxydase, la cholestérol oxydase, la dihydro-orotate oxydase, l'oxalate oxydase, la tyramine oxydase, la L-2-hydroxylate oxydase, la pyridoxine phosphate oxydase, la pyruvate oxydase, la putrescine oxydase, l'hexose oxydase, la lathostérol oxydase et la lysine α-oxydase,

de préférence la glucose oxydase.

5. Système selon la revendication 4, dans lequel le substrat enzymatique contient l'un ou plusieurs, de préférence l'un, des substrats suivants : la L-leucine, la L-méthionine, la L-alanine, l'acétaldéhyde, la purine, l'hypoxanthine, l'éthanolamine, le D-galactose, le lactose, la xanthine, l'acide glycolique, l'acide lactique, le glycérol, la dihydroxyacétone, le glycéroltriphosphate, le glucose, le D-mannose, le D-galactose, l'acide D-glutamique, l'acide orotique, l'acide D-dihydroorotique, l'acide oxalique, la tyramine, la dopamine, l'acide L-2-hydroxyisocapronique, l'acide glycolique, la pyridoxine-5-phosphate, l'acide pyruvique, la putrescine, le D-glucose, le D-galactose, le lathostérol, la L-lysine, la L-ornithine et la L-phénylalanine
de préférence le glucose.

6. Système selon l'une des revendications 3 à 5, dans lequel le système comprend un dosage de type sandwich ou un dosage de type compétitif.

7. Système selon l'une des revendications 3 à 6, dans lequel les moyens de réduction du superoxyde sont ajoutés à une solution contenant la substance avant le dosage.

8. Système selon l'une des revendications 3 à 7, dans lequel le moyen de réduction du superoxyde est la superoxyde dismutase, la superoxyde dismutase étant présente en quantité supérieure à environ 5 μg/ml, de préférence supérieure à environ 10 μg/ml.

9. Système selon l'une des revendications 3 à 8, dans lequel l'enzyme est immobilisée sur un support.

10. Système selon la revendication 9, dans lequel l'enzyme est liée à un anticorps immobilisé sur le support.

11. Méthode pour la détermination de la quantité d'un antigène dans une solution en utilisant un système tel que défini dans la revendication 1, comprenant :

(i) la mise en contact de l'antigène avec un anticorps immobilisé pour cela sur un support ;
(ii) l'élimination de l'antigène libre ;
(iii) la mise en contact de l'antigène lié avec un anticorps marqué par une oxydase capable de se fixer à l'antigène lié ;
(iv) l'élimination de l'anticorps libre marqué par une oxydase ;
(v) l'introduction d'un substrat de l'oxydase, de manière à permettre ainsi le dosage de l'activité enzymatique,

et, optionnellement, la répétition d'au moins une des étapes ci-dessus, au moins une fois, l'oxydase étant capable de catalyser la production de peroxyde à partir du substrat, caractérisé en ce que la méthode contient en plus l'utilisation de moyens, de préférence la superoxyde dismutase, pour réduire les niveaux de superoxyde dans au moins une des étapes (i) à (v).

12. Méthode pour la détermination de la quantité d'un antigène dans une solution en utilisant un système tel que défini dans la revendication 1, comprenant :

(i) la mise en contact de l'antigène et d'une quantité de l'antigène marqué avec une oxydase, avec un anticorps, et cela pour former un complexe ;
(ii) la mise en contact du complexe avec un anticorps immobilisé capable de se fixer au complexe ;
(iii) l'élimination du complexe libre ;
(iv) l'introduction du substrat de l'oxydase de manière à permettre ainsi le dosage de l'activité enzymatique,

et, optionnellement, la répétition d'au moins une des étapes ci-dessus, au moins une fois, l'oxydase étant capable de catalyser la production de peroxyde à partir du substrat, caractérisé en ce que la méthode contient en plus l'utilisation de moyens, de préférence la superoxyde dismutase, pour réduire les niveaux de superoxyde dans au moins une des étapes (i) à (iv).

13. Méthode pour la détermination de la quantité d'un antigène en solution par l'utilisation d'un système tel que défini dans la revendication 1, comprenant :

(i) la mise en contact de l'antigène avec un anticorps immobilisé pour cela sur un support ;
(ii) la mise en contact de l'antigène lié avec un anticorps marqué par une oxydase capable de se fixer à l'antigène lié ;
(iii) l'élimination de l'anticorps libre marqué par une oxydase, de l'antigène libre et du complexe libre (anticorps marqué par une enzyme (/antigène ;
(iv) l'introduction d'un substrat de l'oxydase de manière à permettre ainsi le dosage de l'activité enzymatique,

et, optionnellement, la répétition d'au moins une des étapes ci-dessus, au moins une fois, l'oxydase étant capable de catalyser la production de peroxyde à partir du substrat, caractérisé en ce que la méthode contient en plus l'utilisation de moyens de préférence la superoxyde dismutase, pour réduire les niveaux de superoxyde dans au moins une des étapes (i) à (iv).

14. Méthode selon l'une des revendications 11 à 13, dans laquelle l'oxydase contient une ou plusieurs, de préférence une, des enzymes suivantes : la L-amino-acide oxydase, l'aldéhyde oxydase, l'éthanolamine oxydase, la galactose oxydase, la xanthine oxydase, la glycolate oxydase, la glycérol oxydase, la glycérol-3-phosphate oxydase, la glucose oxydase, la D-glutamate oxydase, la cholestérol oxydase, la dihydro-orotate oxydase, l'oxalate oxydase, la tyramine oxydase, la L-2-hydroxylate oxydase, la pyridoxine phosphate oxydase, la pyruvate oxydase, la putrescine oxydase, l'hexose oxydase, la lathostérol oxydase et la lysine α-oxydase,
de préférence la glucose oxydase.

15. Méthode selon la revendication 14, dans laquelle le substrat de l'enzyme contient l'un ou plusieurs, de préférence l'un, des substrats suivants : la L-leucine, la L-méthionine, la L-alanine, l'acétaldéhyde, la purine, l'hypoxanthine, l'éthanolamine, le D-galactose, le lactose, la xanthine, l'acide glycolique, l'acide lactique, le glycérol, la dihydroxyacétone, le glycéroltriphosphate, le glucose, le D-mannose, le D-galactose, l'acide D-glutamique, l'acide orotique, l'acide D-dihydroorotique, l'acide oxalique, la tyramine, la dopamine, l'acide L-2-hydroxyisocapronique, l'acide glycolique, la pyridoxine-5-phosphate, l'acide pyruvique, la putrescine, le D-glucose, le D-galactose, le lathostérol, la L-lysine, la L-ornithine et la L-phénylalanine
de préférence le glucose.

16. Méthode selon l'une des revendications 11 à 15, dans laquelle les moyens de réduction du superoxyde sont ajoutés avant que le substrat ne soit ajouté.

17. Méthode selon la revendication 16, dans laquelle les moyens de réduction du superoxyde sont ajoutés à la solution contenant l'antigène.

18. Méthode selon l'une des revendications 11 à 17, dans laquelle le moyen de réduction du superoxyde est la superoxyde dismutase, la superoxyde dismutase étant ajoutée en une quantité supérieure à environ 5 μg/ml, de préférence supérieure à environ 10 μg/ml.

19. Méthode selon l'une des revendications 11 à 18, dans laquelle les moyens de réduction du superoxyde sont utilisés dans la préparation du du réactif témoin.

20. Méthode pour la sensibilisation d'un dosage dans laquelle de faibles niveaux de peroxyde d'hydrogène produits par une oxydase à partir d'un substrat non chromogène sont détectés, comprenant l'utilisation de moyens, de préférence la superoxyde dismutase, pour réduire les niveaux de superoxyde.

FIG.1.

FIG.2.

FIG.3.

FIG.4.